# EUROPÄISCHE PATENTSCHRIFT

(11) **EP 2 542 149 B1**
(45) Veröffentlichungstag und Bekanntmachung des Hinweises auf die Patenterteilung: **09.04.2014**
(21) Anmeldenummer: 11708001.0
(22) Anmeldetag: 28.02.2011
(51) Int. Cl.: G01L 1/14, A61B 5/0215, A61B 5/00, A61B 5/03, A61B 5/107, A61B 5/07, H01G 5/16

(54) **IMPLANTIERBARE VORRICHTUNG ZUM ERFASSEN EINER GEFÄSSWANDDEHNUNG**
IMPLANTABLE DEVICE FOR DETECTING A VESSEL WALL EXPANSION
DISPOSITIF IMPLANTABLE POUR LA DÉTERMINATION D'UNE DILATATION D'UNE PAROI VASCULAIRE

(30) Priorität: 05.03.2010 DE 102010010348
(43) Veröffentlichungstag der Anmeldung: 09.01.2013
(73) Patentinhaber: Albert-Ludwigs-Universität Freiburg, 79104 Freiburg (DE)
(72) Erfinder: WOIAS, Peter, 79102 Freiburg (DE); ZENS, Martin, 79098 Freiburg (DE); BINGGER, Philipp, 79106 Freiburg (DE)
(74) Vertreter: Rösler, Uwe
(86) Internationale Anmeldenummer: PCT/EP2011/000974
(87) Internationale Veröffentlichungsnummer: WO 2011/107247

(56) Entgegenhaltungen:
- WO-A2-2005/096348
- US-A- 4 531 267
- US-A- 6 025 725
- US-A1- 2004 133 092
- US-A1- 2008 065 225
- US-A1- 2008 087 069
- US-B2- 7 526 961
- BINGGER P ET AL: "Implantable multi sensor system for in vivo monitoring of cardiovascular parameters", SOLID-STATE SENSORS, ACTUATORS AND MICROSYSTEMS CONFERENCE, 2009. TRANSDUCERS 2009. INTERNATIONAL, IEEE, PISCATAWAY, NJ, USA, 21. Juni 2009 (2009-06-21), Seiten 1469-1472, XP031545732, ISBN: 978-1-4244-4190-7

## Beschreibung

### Technisches Gebiet

Die Erfindung bezieht sich auf eine implantierbare Vorrichtung zum Erfassen einer Dehnung, d.h. einer elastischen Verformung einer intrakorporalen Gefäßwand, bspw. des Magens, der Speiseröhre, von Venen, Arterien etc., mit einer an die Gefäßwand mittel- oder unmittelbar applizierbaren, flächenelastisch ausgebildeten, dielektrisches Polymer enthaltenden Trägerstruktur, die wenigstens eine kapazitive Elektrodenanordnung vorsieht, deren zuordenbare elektrische Kapazität durch eine elastische Verformung der Trägerstruktur beeinflussbar ist. Ferner wird ein Verfahren zur Herstellung einer diesbezüglichen Vorrichtung beschrieben.

### Stand der Technik

Implantierbare Vorrichtungen zur Erfassung von Formänderungen an Gefäßwänden dienen vornehmlich zur intrakorporalen Blutdruckmessung an blutführenden Gefäßen. Hierzu sind weitgehend biegelastische Sensoren bekannt, die auf Basis kapazitiver oder resistiver Dehmessstreifen arbeiten und an der Gefäßaußenwand anbringbar sind ohne dabei die natürliche Gefäßwandverformung zu sehr zu beeinträchtigen.

Aus der DE 10 2005 035 022 A1 ist ein derartiger implantierbarer Blutdrucksensor zu entnehmen, der ein aus elastischem Material bestehendes, das blutführende Gefäß ringförmig umfassendes Mittel vorsieht, dessen Elastizität in etwa der Eigenelastizität der Gefäßwand entspricht, so dass das ringförmige Mittel das Blutdruck bedingte natürliche Verformungsverhalten der Gefäßwand nicht nachhaltig beeinträchtigt. Zur messtechnischen Erfassung des Verformungsverhaltens ist an dem ringförmigen Mittel ein Dehnungsmessstreifen mit kapazitiv oder induktiv wirkenden Elektrodenstrukturen angebracht. Im Falle kapazitiv wirkenden Elektrodenstrukturen befinden sich die als Kondensatorelektroden ausgebildeten Elektrodenstrukturen an sich jeweils gegenüberliegenden Ringreichen, so dass die Kondensatorelektroden das blutführende Gefäß, gleichsam eines Dielektrikums beidseitig begrenzen.

Eine vergleichbare Sensoreinheit ist in der EP 1 635 158 A2 beschrieben, die zur Erfassung der pulsatilen Ausdehnung eines blutführenden Gefäßes wenigstens zwei in einem bandförmigen, zu einem Ring geschlossenen elastischen Trägerelement eingebrachte Kondensatorelektroden vorsieht, deren Plattenabstand durch den Durchmesser des blutführenden intrakorporalen Gefäßes bestimmt wird. Ganz wesentlich ist hier die Anordnung beider Kondensatorelektroden jeweils gegenüberliegend zum blutführenden Gefäß. Im Wege der pulsatilen Ausdehnung des Blutgefäßes ändert sich zugleich der Kondensatorplattenabstand und somit die elektrische Kapazität der Messanordnung. Zur Blutdruckbestimmung und Messdatenerfassung- und übertragung ist zusätzlich eine mit den Kondensatorelektroden verbundene Planarspule zur Ausbildung eines Resonatorschwingkreises vorgesehen, dessen Resonanzfrequenz von der elektrischen Kapazität des drucksensiblen Sensors abhängt. Das von dem Resonanzschwingkreis auslesbare Signal kann mit einer extrakorporal vorgesehenen Empfangseinheit erfasst werden, das zudem ein Maß für die Formänderung des Blutgefäßes und letztlich ein Maß für den Blutdruck darstellt.

Beide vorstehend erläuterten bekannten implantierbaren Blutdruckmesssysteme weisen jeweils aus dünnem Metall, vorzugsweise aus Kupfer oder Gold gefertigte Kondensatorelektrodenflächen auf, die über keine oder nur über eine begrenzte Dehnbarkeiten verfügen. Diesen Nachteil weist auch die in der US 2004/0133092 A1 erläuterte, implantierbare Sensoreinrichtung auf. Um die natürliche Verformbarkeit des Blutgefäßes nicht zu beeinträchtigen gilt es daher die Elektrodenflächen möglichst klein zu wählen, wodurch jedoch die für die Blutdruckerfassung auswertbaren Signalpegel sehr klein werden.

Hinzu kommt, dass bei den bekannten implantierbaren Blutdruckmessanordnungen, die Kondensatorelektrodenflächen relativ zum Blutgefäß einander gegenüber liegen. Sie formen damit einen elektrischen Kondensator, dessen Plattenabstand durch den Durchmesser des Blutgefäßes definiert wird. Das zwischenliegende Dielektrikum umfasst somit auch das Gewebematerial des Blutgefäßes sowie den durch das Blutgefäß hindurch strömenden Blutstrom, dessen Dielektrizitätskonstante aufgrund des pulsativen Strömungsverhaltens als nicht konstant angenommen werden muss. Darüber hinaus ist Blut elektrisch leitfähig, wodurch bei der kapazitiven Messung elektrische Verluste auftreten, die zusätzlich die auswertbaren Messsignalpegel beeinträchtigen. Ein weiterer Nachteil aufgrund der am zu vermessenden Gefäß gegenüber liegend angeordneten Kondensatorelektrodenflächen besteht auch im Auftreten von elektrischen Streufeldern, die aufgrund von Relativbewegungen des zu vermessenden Blutgefäßes zum umgebenden Gewebe auftreten, und die die Messergebnisse ebenso nachhaltig zu beeinträchtigen vermögen.

### Darstellung der Erfindung

Der Erfindung liegt die Aufgabe zugrunde eine implantierbare Vorrichtung zum Erfassen einer Dehnung, d.h. einer elastischen Verformung einer intrakorporalen Gefäßwand mit einer an die Gefäßwand mittel- oder unmittelbar applizierbaren, flächenelastisch ausgebildeten, dielektrisches Polymer enthaltenden Trägerstruktur, die wenigstens eine kapazitive Elektrodenanordnung vorsieht, deren zuordenbare elektrische Kapazität durch eine elastische Verformung der Trägerstruktur beeinflussbar ist, derart weiterzubilden, dass zum einen dafür Sorge getragen werden soll das natürliche Verformungsverhalten der Gefäßwand nicht zu beeinflussen und zum anderen die auswertbare Signalqualität und Signalstärke erheblich zu verbessern. Insbesondere gilt es Maßnahmen zu treffen, um verfälschende Störeinflüsse auf die Messsignale und letztlich auf das Messergebnis, wie beispielsweise durch Auftreten von Streufeldern oder durch zeitlich sich ändernde Dielektrizitätseigenschaften des zwischen den Elektrodenflächen vorhandenen Dielektrikums etc., zu vermeiden.

Die Lösung der der Erfindung zugrunde liegenden Aufgabe ist im Anspruch 1 und 6 angegeben. Ein lösungsgemäßes Verfahren zur Herstellung der implantierbaren Vorrichtung ist Gegenstand des Anspruches 13. Den Erfindungsgedanken vorteilhaft weiterbildende Merkmale sind Gegenstand der Unteransprüche sowie der weiteren Beschreibung insbesondere unter Bezugnahme auf die Ausführungsbeispiele zu entnehmen.

Um das natürliche pulsatile Verformungsverhalten intrakorporaler Gefäßwände, die aus einem sehr weichen und hochflexiblen Gewebematerial bestehen, das typischerweise über ein E-Modul von 1 MPa und einem Dehnvermögen von 10 % und mehr verfügen, ohne jegliche Beeinflussung, d.h. ohne Einschnürung sowie lokaler Flächenversteifung der sich elastisch verformenden Gefäßwand, messtechnisch zu erfassen, wird ein aus hochelastischen und flexiblen Materialien bestehender, auf dem kapazitiven Messprinzip beruhender Dehnungsmesssensor vorgeschlagen, der keinen nennenswerten Störeinflüssen unterliegt und darüber hinaus die Möglichkeit vielgestaltiger Ausführungsformen bietet.

Lösungsgemäß zeichnet sich die implantierbare Vorrichtung zum Erfassen einer Dehnung, d.h. einer elastischen Verformung einer intrakorporalen Gefäßwand mit einer an die Gefäßwand mittel- oder unmittelbar applizierbaren, flächenelastisch ausgebildeten, dielektrisches Polymer enthaltende Trägerstruktur, die wenigstens eine kapazitive Elektrodenanordnung vorsieht, deren zuordenbare elektrische Kapazität durch eine elastische Verformung der Trägerstruktur beeinflussbar ist dadurch aus, dass die Elektrodenanordnung wenigstens zwei jeweils aus einem elektrisch leitfähigen Polymer bestehende Elektroden vorsieht, die jeweils wenigstens einseitig einen die elektrische Kapazität der Elektrodenanordnung beeinflussenden, vollständig mit dem dielektrischen Polymer der Trägerstruktur gefüllten Zwischenraum begrenzen. Die Trägerstruktur ist flächig ausgebildet und weist eine Trägerstrukturober- und -unterseite auf. In einer ersten lösungsgemäßen Variante ist auf der Trägerstrukturober- und -unterseite jeweils eine Elektrode in Form einer elektrisch leitfähigen strukturierten oder unstrukturierten Polymerschicht aufgebracht. In einer zweiten lösungsgemäßen Variante sind in einer sich zwischen der Trägerstrukturober- und -unterseite erstreckenden und parallel zu der Trägerstrukturober- und -unterseite orientierten ersten Ebene die wenigstens zwei Elektroden vorgesehen, die in dieser Ebene lateral zueinander beabstandet und vollständig von dem dielektrischen Polymer der Trägerstruktur umgeben sind.

Unter dem Begriff "Gefäßwand" ist grundsätzlich eine intrakorporale, aus biologischem Gewebe bestehende Wand zu verstehen, wie beispielsweise die Gefäßwand von Arterien, Venen, Kapillaren oder Lymphgefäßen. Im lösungsgemäßen Zusammenhang sollen aber auch Organwände, wie die Magenwand, die Speiseröhre, Darmwand o. ä. mit umfasst sein.

Im Unterschied zu den bekannten, vergleichbaren implantierbaren Messvorrichtungen sieht die lösungsgemäße Vorrichtung eine, vorzugsweise vollständig aus elektrisch leitfähigem Polymer bestehende Elektrodenanordnung vor, die sowohl aufgrund ihrer materialinherenten flächenelastischen Eigenschaften als auch ihrer Form- und Größenwahl keine oder nur unwesentliche die natürlichen Dehnungseigenschaften der Gefäßwand beeinträchtigende Wirkung zeigt. Insbesondere zeichnet sich die als Kondensatorelektroden ausgebildete Elektrodenanordnung dadurch aus, dass das die Kapazität der Elektrodenanordnung bestimmende Dielektrikum ausschließlich durch die Materialwahl der Trägerstruktur bestimmt ist, so dass es möglich ist, die Messvorrichtung bereits außerhalb des Körpers, d.h. "ex-vivo" zu kalibrieren. Potentielle Messfehler, die von variablen Dielektrizitätskonstanten herrühren, wie beispielsweise bedingt durch einen dynamischen Blutstrom, der zwischen den Kondensatorelektroden hindurchströmt, können auf diese Weise bewusst vermieden werden.

Zur Erläuterung des Aufbaus einer lösungsgemäß ausgebildeten implantierbaren Vorrichtung, sowie deren vorteilhafte Weiterbildungen sei auf die nachstehenden Beschreibungen verwiesen, die auf die figürlich dargestellten konkreten Ausführungsbeispiele Bezug nehmen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a-d: Mehrseitendarstellungen eines ersten lösungsgemäß ausgebildeten Ausführungsbeispiels,
- Fig. 2a-d: lösungsgemäß ausgebildetes Ausführungsbeispiel mit strukturierter Elektrodenanordnung,
- Fig. 3a-d: Mehrseitenansicht eines lösungsgemäß ausgebildeten Ausführungsbeispiels mit Interdigitalelektrodenanordnung,
- Fig. 4a-d: Mehrseitenansicht einer Erweiterungsvariante zu Figur 3,
- Fig. 5: Querschnitt durch ein Ausführungsbeispiel mit zusätzlicher Abschirmung,
- Fig. 6: weitere Variante mit externer Abschirmung und
- Fig. 7: Ausführungsbeispiel mit Abschirmelektroden.

### Wege zur Ausführung der Erfindung, gewerbliche Verwendbarkeit

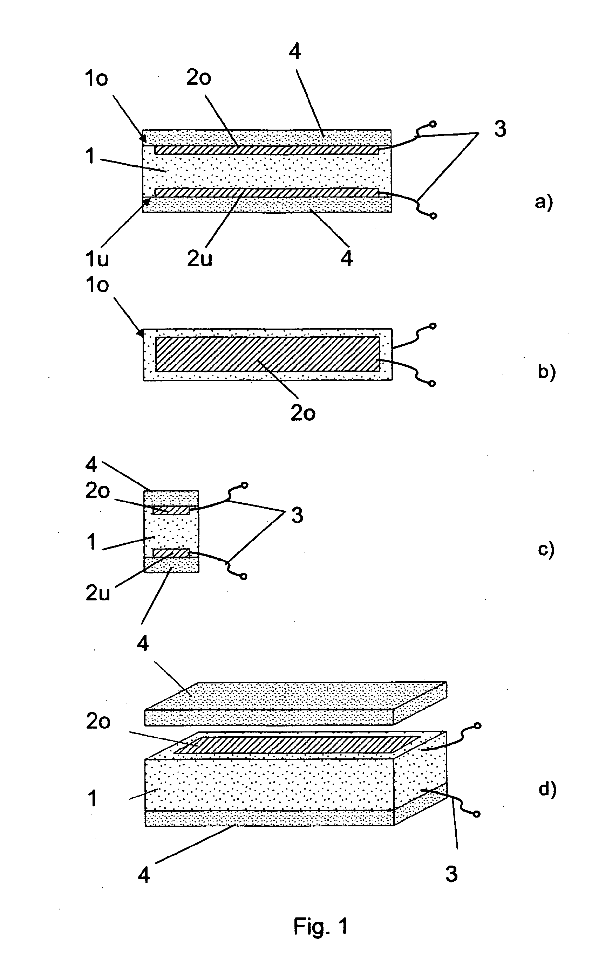

In den Figuren 1a-d ist das Grundprinzip für den Aufbau einer lösungsgemäß ausgebildeten, implantierbaren Vorrichtung in einer Mehrseitendarstellung illustriert. Figur 1a zeigt einen Längsschnitt durch eine lösungsgemäß ausgebildete Vorrichtung, die eine Trägerstruktur 1 vorsieht, die aus einem dielektrischen Polymer, vorzugsweise aus einem hochelastischen Silikon besteht, dass über eine Eigenelastizität verfügt, die mit der Elastizität einer intrakorporalen Gefäßwand vergleichbar ist, d.h. über ein E-Modul von etwa 1 MPa verfügt. Derartige Polymermaterialien sind dem Fachmann bekannt und industriell herstellbar. Die Trägerstruktur 1 ist flächig ausgebildet und weist eine Trägerstrukturoberseite 1o und eine Trägerstrukturunterseite 1u auf. Typischerweise besitzt die Trägerstruktur eine Dicke von einigen 10 bis einigen 100 µm, beispielsweise 50 µm bis 400 µm. Sowohl an der Strukturoberseite 1 o als auch an der Strukturunterseite 1 u ist jeweils eine aus einer elektrisch leitfähigen Polymerschicht bestehende Elektrode 2o, 2u angebracht. Beide Elektroden 2o, 2u sind mit elektrischen Kontaktierungen 3, beispielsweise in Form dünner Drähte verbunden. Die Elektroden 2o, 2u sind im Ausführungsbeispiel gemäß Figur 1 flächig, streifenförmig ausgebildet und jeweils an der Trägerstrukturoberseite 1o sowie Trägerstrukturunterseite 1u eingebettet, d.h. jeweils auch seitlich von einem umlaufenden Trägerstrukturkragen umgeben. Figur 1 b zeigt in einer Schnittdarstellung die Draufsicht auf die Trägerstrukturoberseite 1 o mit der darin eingebetteten oberen Elektrodenschicht 2o.

Zu Zwecken einer elektrischen Isolation und Kapselung der aus elektrisch leitfähigem Polymer bestehenden Elektroden 2o, 2u ist zumindest an der Ober- und Unterseite der Trägerstruktur 1 jeweils eine Isolationsschicht 4 aufgebracht, die aus dem gleichen flächenelastischen, dielektrischen Polymer der Trägerstruktur bestehen kann oder aus einem von der Trägerstruktur unterschiedlichen Polymermaterial, das jedoch über vergleichbare Elastizitätseigenschaften verfügt. Nur aus Gründen einer besseren Veranschaulichung ist in der Draufsichtdarstellung gemäß Figur 1 b die obere Isolationsschicht 4 weggelassen worden.

Die Querschnittsdarstellung gemäß Figur 1c verdeutlicht die Anbringung jeweils der oberen und unteren elektrisch leitfähigen Polymerelektrodenschicht 2o und 2u, die jeweils auch seitlich von der Trägerstruktur 1 umschlossen sind. Die Elektroden 2o, 2u schließen einen gegenseitigen Abstand ein, der vollständig mit dem hochelastischem Polymer der Trägerstruktur 1 ausgefüllt ist. Somit trägt ausschließlich das Polymermaterial der Trägerstruktur als Dielektrikum zur Kapazität der Elektrodenanordnung bei. Bedingt durch eine mechanische Verformung der in Figur 1 illustrierten Elektrodenanordnung samt Trägerstruktur und der darauf jeweils aufgebrachten Isolierschichten 4 kommt es zu einer Änderung des Elektrodenabstandes zwischen den beiden leitfähigen Polymerschichten, wodurch eine messbare Kapazitätsänderung die Folge ist. Zur Kapazitätsmessung wird zwischen beiden Elektroden 2o, 2u eine elektrische Potentialdifferenz angelegt, die über die beiden Kontaktdrähte 3 applizierbar ist.

Eine Möglichkeit zur Auslesung des Sensors besteht darin, die Kontaktdrähte 3 mit einer ebenfalls implantierbaren elektrischen Ausleseeinheit mit integrierter Energiequelle, bspw. Batterie, zu verbinden, oder die Energieversorgung durch Bereitstellen einer extrakorporal drahtlosen Energieversorgungstechnik zu bewerkstelligen. Hierzu sind die Elektroden 2o, 2u über die Kontaktdrähte 3 mit einer geeignet ausgebildeten Induktivität zu verbinden, die gleichsam der Elektroden 2o, 2u innerhalb der Trägerstruktur 1 implementiert bzw. eingebettet ist. Mit Hilfe einer extrakorporal vorgesehenen Spule kann durch induktive Kopplung elektrische Energie in die intrakorporal innerhalb der Trägerstruktur 1 vorgesehene Induktivität eingekoppelt werden.

Die verformungsbedingte Kapazitätsänderung kann gleichfalls mit Hilfe der extrakorporal vorgesehenen Energieversorgungseinheit, die typischerweise eine Antenneneinheit darstellt, erfasst werden, zumal die innerhalb der Trägerstruktur und mit den kapazitiven Elektroden 2o, 2u verbundene Induktivität einen Schwingkreis bildet, dessen Resonanzfrequenz wesentlich durch die Kapazität der kapazitiven Elektrodenanordnung bestimmt ist. Eine Kapazitätsänderung spiegelt sich in einer Änderung der Resonanzfrequenz des Schwingkreises wieder, die wiederum mit Hilfe der extrakorporal vorgesehenen Antenneneinheit erfassbar ist. Eine derartige drahtlose Messsignalerfassung ist dem Fachmann auch als GRID-Dipping bekannt.

Figur 1d zeigt in perspektivischer Darstellung die lösungsgemäß ausgebildete implantierbare Vorrichtung, bei der zur Sichtbarmachung der oberen Elektrode 2o die obere Isolatorschicht 4 leicht abgehoben dargestellt ist. Der lediglich abschnittsweise in Figur 1 dargestellte Schichtverbund aus den Isolatorschichten 4, den Elektrodenschichten 20, 2u und der dazwischen liegenden Trägerstruktur 1 ist typischerweise als Längsstreifen mit einer Streifenlänge ausgebildet, der einmalig bündig um den Außenumfang eines blutführenden Gefäßes angelegt werden kann. Zur Befestigung des streifenförmig ausgebildeten Schichtverbundes an der intrakorporalen Gefäßwand werden vorzugsweise beide Streifenbereichsenden mittels Klebe-, Schweiß-, Klemm- oder Nähtechnik aneinander fest gefügt.

Die Herstellung des in Figur 1 illustrierten Blutdrucksensors erfolgt vorzugsweise schichtförmig, indem auf einer ersten, unteren Dielektrikumschicht 4 eine weitgehend unstrukturierte, vorzugsweise rechteckförmig ausgebildete Elektrodenfläche 2u aufgebracht wird, indem ein elektrisch leitfähiges Polymer bspw. im fließfähigen Zustand oder in einer vorgefertigter festen Folienform auf die Oberfläche des Dielektrikums 4 aufgebracht wird. Nachfolgend wird das elektrisch isolierende Dielektrikum der Trägerstruktur aufgebracht, wobei die untere Elektrodenfläche 2u vollständig sowohl seitlich als auch an ihrer freien Oberfläche von dem Trägerstrukturmaterial umgossen bzw. umschlossen wird. Schließlich erfolgt das Aufbringen der oberen Elektrode 2o und der darauf aufgebrachten Dielektrikumschicht 4. Wie bereits vorstehend erläutert beträgt der Abstand zwischen beiden Elektrodenschichten 2o, 2u, d.h. die Dicke des Dielektrikums innerhalb der Trägerstruktur 1 etwa 50 µm bis 400 µm, vorzugsweise jedoch 100 µm. Die Dicken der Elektrodenschichten 2o, 2u sind in etwa gleich groß oder dünner dimensioniert als die Trägerstrukturdicke. In vorteilhafter Weise sind beide Elektroden 2o, 2u deckungsgleich vertikal übereinander angeordnet, um einen maximalen Überdeckungsgrad zu realisieren. Gleichwohl ist es jedoch auch möglich, die Elektroden 2o, 2u in geeigneter Weise versetzt zueinander anzuordnen oder an diesen zusätzliche Kontaktierungselemente vorzusehen, um mögliche Feinabstimmungen zur Anpassung des Resonanzschwingkreises vornehmen zu können.

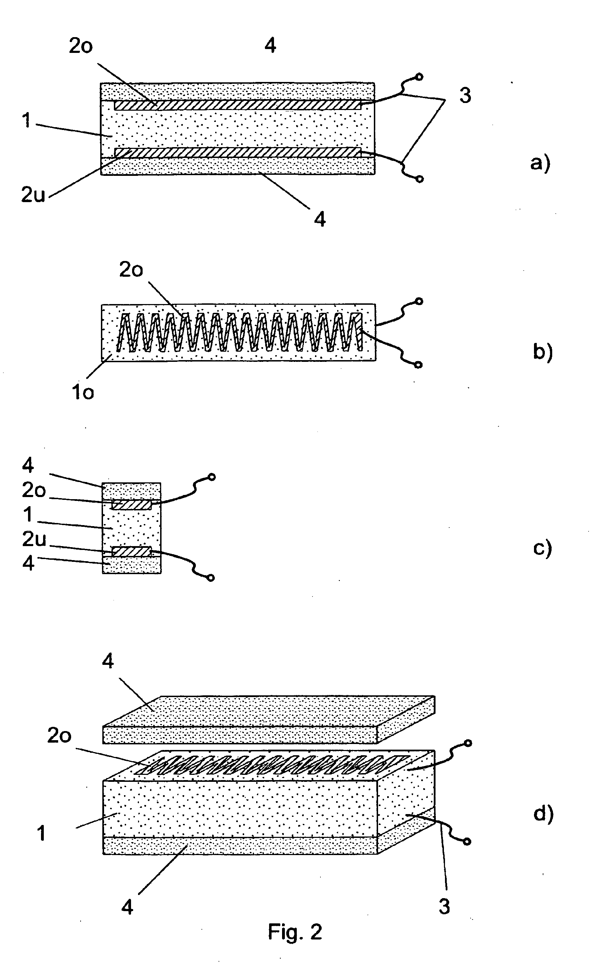

Eine in Figur 2a-d illustrierte zweite Ausführungsform für eine lösungsgemäß implantierbar ausgebildete Vorrichtung verfügt grundsätzlich über die gleichen Komponenten wie das in Figur 1 illustrierte Ausführungsbeispiel - es werden die gleichen Bezugszeichen für bereits beschriebenen gleiche Komponenten verwendet -, jedoch sind in diesem Fall die Elektrodenflächen 2o, 2u nicht in Form unstrukturierter rechteckförmig ausgebildeter Flächenelektroden, sondern strukturiert in Form z.B. eines Zick-Zack-Musters (siehe hierzu Draufsichtdarstellung gemäß Figur 2b) ausgebildet. Durch die Zick-Zack-förmige Strukturierung sowohl der oberen als auch der unteren Elektrode 2o, 2u wird eine wesentlich höhere Flächenelastizität der kapazitiv wirkenden Elektroden 2o, 2u erreicht im Vergleich zur vorstehend beschriebenen, unstrukturierten Ausführungsform der Elektroden 2o, 2u. In vorteilhafter Weise sind die strukturierten Elektroden 2o, 2u in orthogonale Projektion auf die Trägerstrukturober- und -unterseite 1o, 1 u möglichst deckungsgleich angeordnet. Wie im Falle des Ausführungsbeispiels in Figur 1 befindet sich zwischen beiden strukturierten Elektroden 2o, 2u ausschließlich dielektrisches Polymer, vorzugsweise elastisches dehnfähiges Silikon, das das Dielektrikum für die kapazitive Elektrodenanordnung darstellt.

Die Strukturierung der Elektroden 2o, 2u kann mit Hilfe einer Laserbearbeitung vorgenommen werden, beispielsweise können unstrukturierte, rechteckförmig ausgebildete elektrisch leitfähige Polymerschichten, die bereist auf der Trägerstruktur aufgebracht sind, mit Hilfe eines vom Material des elektrisch leitfähigen Polymers absorbierenden Lasers bearbeitet werden, der das dielektrische Trägerstrukturmaterial weitgehend absorptionsfrei zu durchdringen vermag. Mit Hilfe des Laserbearbeitungsverfahrens können nicht nur nahezu beliebige zweidimensionale Strukturen aus einer homogen applizierten, elektrisch leitfähigen Polymerschicht herausgearbeitet werden, zusätzlich ist es möglich, die Begrenzungskanten der strukturierten Elektrodenschicht in Bezug auf ihre Flankensteilheit entsprechend zu formen. Auf diese Weise können die sich zwischen beiden durch das Dielektrikum der Trägerstruktur beabstandeten Elektroden ausbildenden elektrischen Felder in vorteilhafter Weise unter Vermeidung störender Streufelder ausgebildet bzw. optimiert werden.

Neben einer verbesserten Dehnbarkeit vermeiden strukturierte Elektroden, vorzugsweise in der aus den Figuren 2b und d entnehmbaren Weise, bei großen Verformungen Brüche oder Rissbildungen und verhelfen somit dem Sensor zu einergrößeren Robustheit und längeren Lebensdauer.

In den beiden vorstehend beschriebenen Ausführungsformen befinden sich die aus elektrisch leitfähigem Polymer bestehenden Elektroden 2o, 2u in zwei unterschiedlichen Parallelebenen innerhalb der Trägerstruktur 1, im weiteren werden Ausführungsformen beschrieben, in denen beide Elektroden in einer einzigen Ebene angeordnet sind und somit eine deutlich dickenreduziertere Ausbildung der implantierbaren Vorrichtung ermöglichen.

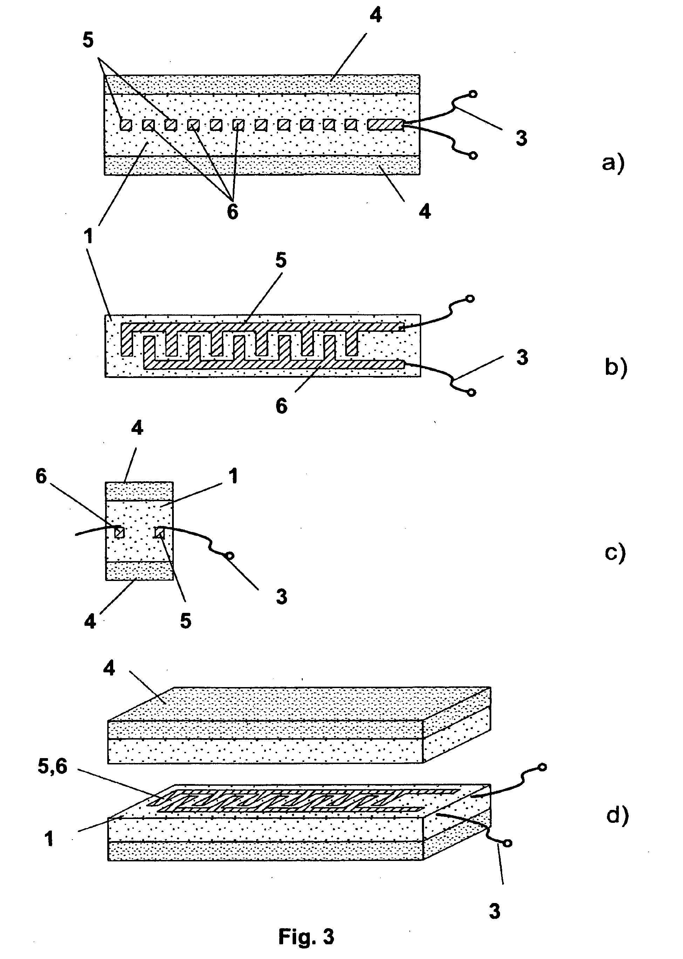

In den Figuren 3a bis 3d ist gleichsam den vorstehenden Figuren 1 und 2 eine Mehrseitenansichtdarstellung von einer implantierbaren Vorrichtung gezeigt, bei der die aus elektrischem Polymer bestehenden Elektroden als Interdigitalstrukturelektroden ausgebildet sind. Dies geht insbesondere aus der Draufsichtdarstellung gemäß Figur 3b hervor, die eine mittlere Schnittebene durch die Trägerstruktur 1 zeigt, in der zwei als Interdigitalelektroden ausgebildete Elektroden 5, 6 angeordnet bzw. eingebettet sind. Figur 3a zeigt einen entsprechenden Längsschnitt durch die implantierbare Vorrichtung mit beidseitig auf der Trägerstrukturober- und unterseite 1o, 1u aufgebrachten Isolatorschichten 4. Figur 3c zeigt einen entsprechenden Querschnitt durch die Vorrichtung. Figur 3d illustriert eine perspektivische Gesamtschau der schichtförmig aufgebauten implantierbaren Vorrichtung, bei der zur Visualisierung der Interdigitalelektrodenanordnung 5, 6 der Schichtaufbau in zwei einander vertikal beabstandete Hälften dargestellt ist.

Die aus elektrisch leitfähigem Polymer bestehenden und in Form einer Interdigitalstruktur ausgebildeten Elektroden 5, 6 weisen jeweils sich gegenseitig berührungslos ineinander greifende Fingerstrukturen innerhalb einer Ebene auf. Der Raum zwischen den in der Ebene jeweils gegenüber liegenden Interdigitalelektrodenstrukturen ist mit dem hochelastischem polymeren Trägerstrukturmaterial, vorzugsweise Silikon vollständig gefüllt, das als Dielektrikum zwischen den Elektroden dient. Bei Applikation einer elektrischen Potentialdifferenz zwischen beiden Elektrodenstrukturen 5,6 bilden sich zwischen den sich unmittelbar gegenüber liegenden Elektrodenflächen nicht nur elektrische Feldlinien aus die senkrecht auf den Elektrodenfläche stehen, hinzukommen elliptisch geformte Feldlinienverläufe zwischen den Fingerelektrodenstrukturen die in die Trägerstruktur 1 ober- und unterhalb der Elektrodenebene 5,6 verlaufen, die einen wesentlichen Beitrag zur messbaren Kapazität liefern. Durch eine mechanische Verformung des in Figur 3 illustrierten Sensors kommt es zu einer Änderung des Abstandes zwischen den einzelnen Elektrodenstrukturfingern der Interdigitalelektrodenstruktur, wodurch sich die Kapazität der Elektrodenanordnung höchst sensibel zu ändern vermag, die gemessen werden kann.

Die aus der Bilddarstellung in Figur 3b und 3d entnehmbare Interdigitalelektrodenanordnung ist lediglich als stilisierte schematische Wiedergabe einer klassischen Interdigitalelektrodenstruktur anzusehen. Mit Hilfe der bereits erwähnten Laserbearbeitung können sehr filigrane Interdigitalelektrodenstrukturen aus flächig abgeschiedenen Polymerschichten geformt werden, die elastizitätsoptimiert ausgebildet sind. Eine mögliche Ausführungsform derartiger elastizitätsoptimierter Elektrodenstrukturen stellen jeweils Hufeisen-förmig ausgebildete Leiterbahnen dar, die durch Aneinanderreihen von 120° geschwungenen Hufeisenpaaren gebildet werden.

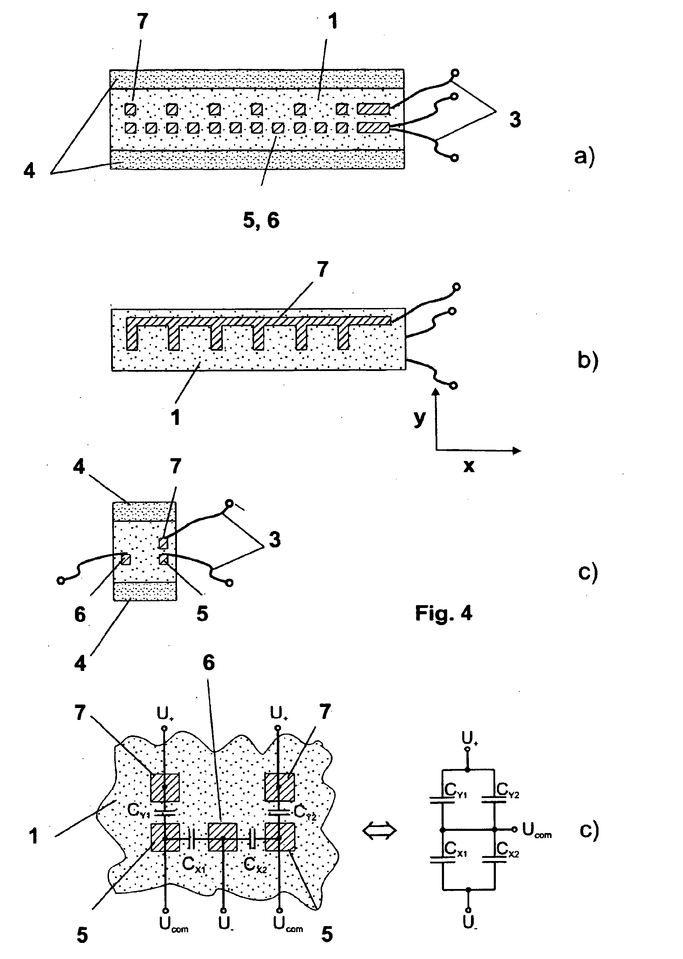

Eine weitere Ausführungsform für eine implantierbare Vorrichtung mit einer Interdigitalelektrodenstrukturanordnung ist in den Figuren 4a-d dargestellt, die im Vergleich zum Ausführungsbeispiel gemäß Figur 3 über eine weitere Interdigitaltelelektrode 7 verfügt, die in einer zweiten Ebene innerhalb der Trägerstruktur 1 angeordnet ist, die parallel zur ersten Ebene orientiert ist, in der die in Figur 3 illustrierte Interdigitalelektrodenstrukturanordnung 5,6 platziert ist. Im Besonderen ist die weitere Interdigitalelektrodenstruktur 7 parallel zur Interdigitalelektrodenstruktur 5 angeordnet. Gleichsam den vorstehenden Figuren ist der Figur 4a eine Längsschnittdarstellung, 4b eine Draufsichtdarstellung sowie Figur 4c eine Querschnittsdarstellung durch die Elektrodenanordnung dargestellt.

Durch die zusätzliche in der zweiten Ebene vorgesehene Interdigitalelektrodenstruktur 7 ist ein sogenannter Differentialkondensator realisiert. Mit dem Design des Differentialkondensators lassen sich Störungen insbesondere alle Gleichtaktstörungen wie z.B. parasitäre Kapazitäten, Offsetfehler, Betriebsspannungs- und Temperatureinflüsse effektiv unterdrücken. Zudem erhöht sich die Sensitivität des Sensors für Verformungen sowohl in der Längs-, d.h. x-Richtung als auch in der orthogonal zur Längsrichtung orientierten Raumrichtung y. Das Prinzip eines Differentialkondensators sei am Ersatzschaltbild gemäß Figur 4d erläutert: In Figur 4d linke Darstellung ist ein Ausschnitt des Längsschnitt gemäß Figur 4a illustriert, aus dem die Interdigitalelektrodenanordnung 5, 6 sowie die in der zweiten Ebene dazu separat angeordnete Interdigitalelektrodenstruktur 7 zu entnehmen sind. So sei angenommen, dass die Trägerstruktur 1 in der linken Bilddarstellung gemäß Figur 4d eine Streckung in x-Richtung erfährt, wodurch die Abstände zwischen den Interdigitalelektroden 5,6 vergrößert werden, wodurch sich die Kapazitäten Cₓ₁ und Cₓ₂ jeweils verringern. Zugleich erfährt die Trägerstruktur 1 in y-Richtung durch die Querkontraktion eine Kompression, wodurch die Abstände zwischen den Interdigitalelektrodenstrukturen 5 und 7 geringer werden und sich somit die Kapazitäten C_{y1} und C_{y2} entsprechend gegensinnig zu Cₓ₁ und Cₓ₂ vergrößern. Mit einem entsprechenden Design lassen sich die Kapazitäten und die Kapazitätsänderungen gleich dimensionieren. Mit Hilfe des in der rechten Bilddarstellung gemäß Figur 4d veranschaulichten Ersatzschaltbild erhöht sich die Selektivität des Sensors bei dieser Messweise sowohl bei Verformungsänderungen in x- als auch in y-Richtung. Zudem wir eine effektive Störunterdrückung erreicht.

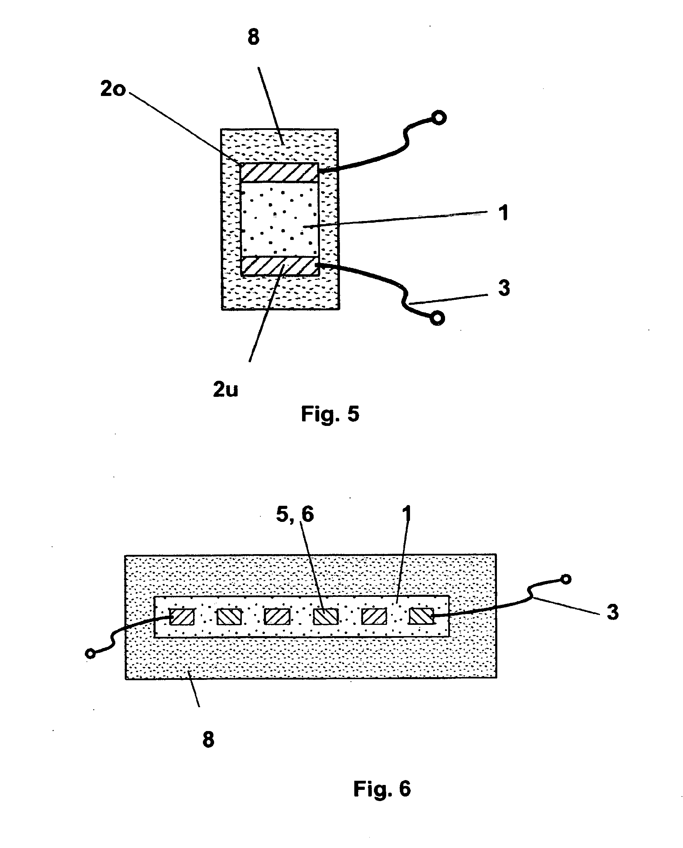

Kapazitive Sensoren erzeugen grundsätzlich je nach ihrem konstruktiven Aufbau elektromagnetische Streufelder, die, je nach Ausprägung in das räumliche Umfeld der Kondensatoranordnung hineinreichen kann. Ändern sich die elektrischen bzw. dielektrischen Eigenschaften des Umfeldes können die im Umfeld wirksamen Streufelder letztlich auf die Kapazität des Sensors rückwirken und diese beeinflussen. Gilt es kapazitive Messungen mit nur geringen Signalpegeln, wie dies bei der lösungsgemäßen Vorrichtung der Fall ist, durchzuführen, so ist es wünschenswert jegliche störenden, die Messergebnisse beeinflussenden Fehlerquellen zu unterdrücken. Insbesondere gilt es die Sensorsignalverfälschung aufgrund von Streufeldern und deren umgebungsbedingten Rückkopplung auf das Messsignal zu minimieren. Eine Möglichkeit diesen Störeffekt zu reduzieren besteht in einer gezielten Fokussierung der sich zwischen den Elektrodenstrukturen ausbildenden elektrischen Feldlinien auf den Bereich zwischen den Elektroden. So ist es grundsätzlich bekannt, dass elektrische Feldlinien in einem dielektrischen Material mit hoher Dielektrizitätskonstante besser "geführt" werden als in einem Material mit geringerer Dielektrizitätskonstante. Dieser Effekt wird in einem bevorzugten Ausführungsbeispiel der lösungsgemäßen implantierbaren Vorrichtung genutzt, indem die Elektrodenstrukturen mit den zwischen den Elektroden eingebrachten dielektrischem Polymermaterial der Trägerstruktur gesamtheitlich von einem Material umgeben werden, das eine höhere Dielektrizitätskonstante aufweist als das im weiteren Umfeld vorhandene Material. Auf diese Weise wird das elektrische Feld auf einen definierten Raum um die Elektrodenanordnung lokalisiert, wodurch der Einfluss möglicher mit der Umgebung in Wechselwirkung tretender Streueffekte reduziert wird. Zur Illustration eines diesbezüglichen bevorzugten Ausführungsbeispiels sei auf Figur 5 hingewiesen, die einen Querschnitt in der lösungsgemäß ausgebildeten Vorrichtung zeigt. An der Trägerstrukturoberseite sowie auch Trägerstrukturunterseite sind jeweils zwei Elektroden 2o, 2u angebracht, gleichsam dem Ausführungsbeispiel in Figur 1, die zusätzlich mit einer hochelastischen dielektrischen Polymerschicht 8 umgeben sind, deren zuordenbare Dielektrizitätskonstante geringer ist als die Dielektrizitätskonstante des dielektrischen Polymers der Trägerstruktur 1. Eine vergleichbare Ummantelung kann auch eine Vorrichtung mit Interdigitalelektrodenstruktur gemäß Figur 6 erhalten, die vollständig mit einer Isolatorschicht 8 umgeben ist, deren Dielektrizitätskonstante geringer ist als die Dielektrizitätskonstante des Dielektrikums der Trägerstruktur 1.

Zusätzlich kann die Ausbreitung von Streufeldern durch eine geeignete Anpassung der Strukturgrößen der einzelnen Komponenten der Vorrichtung reduziert werden. Wählt man beispielsweise den Elektrodenabstand zwischen zwei Elektroden klein gegenüber ihrer flächigen bzw. lateralen Ausdehnung, so bleibt das elektrische Feld aufgrund des geringen Elektrodenabstandes im wesentlichen innerhalb der Kondensatorstruktur fokussiert und vermag nicht in das Umfeld zu streuen.

Eine weitere Möglichkeit Störeinflüsse zu reduzieren besteht darin, die Elektrodenstrukturen vollständig oder partiell mit extra vorzusehenden Abschirmelektroden äußerlich zu umgeben. Mit Hilfe derartiger äußerlich angebrachter Abschirmelektroden, die auf ein definiertes elektrisches Potential zu legen sind, werden die von den kapazitiven Elektroden ausgehenden Streufeldern regelrecht daran gehindert in den die Sensoreinheit umgebenden Raumbereich "einzudringen". In der gleichen Weise können externe Störfelder von außen nicht in die kapazitive Sensorstruktur "eindringen".

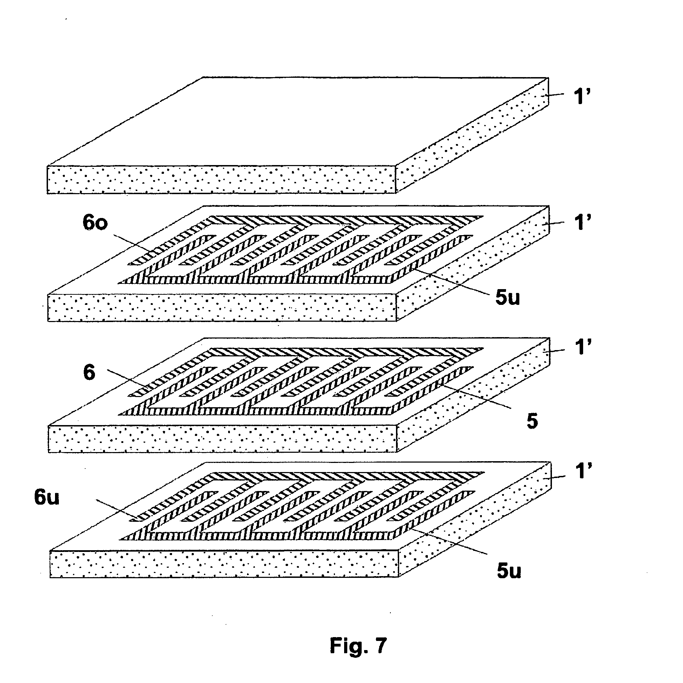

Um die Eigenelastizität der Sensorvorrichtung nicht signifikant zu beeinträchtigen werden Abschirmelektroden lediglich dort lokal vorgesehen, wo eine mögliche Streufeldabstrahlung von den Elektrodenstrukturen maximal ist. Vorzugsweise werden die Abschirmelektroden in Form einer Gitterstruktur ausgeführt, so dass die elastische Verformbarkeit der gesamten Sensorvorrichtung im Wesentlichen erhalten bleibt. Im Falle von kapazitiven Interdigitalelektrodenstrukturen, wie dies beim Ausführungsbeispiel gemäß der Figur 3 und 4 der Fall ist, können die Abschirmelektroden in der gleichen Form strukturiert sein wie die Interdigitalelekroden selbst. Ein derartiges Ausführungsbeispiel ist aus Figur 7 zu entnehmen, das zu besseren Illustration in Schichten dargestellt ist, die zusammenzufügen sind.

So ist für jede Interdigitalelektrode 5, 6 eine eigene Abschirmelektrode 5o, 5u, 6o, 6u vorgesehen, welche dieselbe Grundform wie die Interdigitalelektrode 5, 6 selbst aufweist und, getrennt durch eine isolierende Trägerstrukturzwischenschicht 1', oberhalb bzw. unterhalb von dieser angebracht wird. Dadurch wird die Streukapazität zwischen den Interdigitalelektroden 5, 6 und den Abschirmelektroden 5o, 5u. 6o. 6u möglichst weitgehend minimiert, insbesondere dann, wenn das Potential jeder Abschirmelektrode 5u, 5o, 6u, 6o dem Potential der jeweils zugehörigen Interdigitalelektrode 5, 6 nachgeführt wird.

Selbstverständlich ist es jedoch auch möglich, die Abschirmelektroden unterschiedlich von Form und Größe der kapazitiven Elektrodenanordnung auszubilden und anzuordnen.

Eine nicht weiter illustrierte Ausführungsform basiert gleichfalls auf einer Interdigitalstruktur basiert, die sich jedoch durch ein besonders hohes Aspektverhältnis der Fingerstrukturen auszeichnet. Hierbei setzt sich die zu messende Kapazität hauptsächlich aus dem parallelen elektrischen Feld zusammen, welches sich zwischen zwei gegenüberliegenden Elektrodenstrukturen ausbildet. Fertigungstechnisch lässt sich ein solcher kapazitiver Dehnungsmessstreifen derart konstruieren, dass ein Streifen des hochelastischen Silikonwerkstoffes beidseitig mit dem elektrisch leitfähigen Polymer beschichtet wird. Die Beschichtung kann beispielsweise in Rakeltechnik oder durch Bedampfen erfolgen. Anschließend wird der beschichtete Streifen mäanderförmig aufgerollt und mit dem hochelastischen Silikonwerkstoff vergossen. Durch Kontaktierung der leitfähigen Schichten und anlegen einer Potentialdifferenz kann auch hier eine Kapazität messtechnisch erfasst werden. Bedingt durch mechanische Verformung kommt es bei dieser Anordnung zu einer Änderung des Raumes zwischen den leitfähigen Schichten. Dieser Raumunterschied resultiert in einer Änderung der Kapazität, welche eine messtechnisch erfassbare Größe darstellt.

Wie bereist erwähnt erlaubt das lösungsgemäße kapazitive Sensorprinzip eine drahtlose Auslesung des elektrischen Messsignals mit Hilfe des bekannten sogenannten "Grid Dipping"-Prinzips. Dabei wird die Sensorkapazität der Elektrodenanordnung mit einer elektrischen Induktivität zu einem elektrischen Schwingkreis verbunden. Die Induktivität ist konstruktiv so gestaltet, dass ein elektromagnetisches Wechselfeld, das von außen von einem Sender eingestrahlt wird, einen elektrischen Wechselstrom im Schwingkreis induzieren kann. Wenn die Frequenz des äußeren Wechselfeldes der elektrischen Resonanzfrequenz des Schwingkreises entspricht, dann gerät der Schwingkreis in elektrische Resonanz und entnimmt dabei dem äußeren Wechselfeld ein Maximum an Feldenergie. Diese Abnahme der Feldenergie kann vom Sender des äußeren Wechselfeldes erkannt werden. Wenn nun der äußere Sender ein Wechselfeld mit zeitlich variabler Frequenz aussendet, dann wird er ein selektives Einbrechen der Feldenergie bei der Resonanzfrequenz des Schwingkreises erkennen. Dies erlaubt, dessen Resonanzfrequenz von außen zu bestimmen. Die Resonanzfrequenz eines Schwingkreises aus der kapazitiven Elektrodenanordnung und der Induktivität hängt nach bekannten physikalischen Gesetzmäßigkeiten vom konstanten Induktivitätswert der Spule und der variablen Kapazität der Elektrodenanordnung ab. Somit ist, über die detektierte variable Resonanzfrequenz, die Kapazität drahtlos erfassbar.

In einer praktischen Ausführungsform wird die Induktivität direkt in der Trägerstruktur integriert. Die Verbindung mit der Sensorkapazität erfolgt ebenfalls innerhalb der Trägerstruktur. Auf diese Weise können alle elektrischen Leitungen innerhalb der Trägerstruktur geschützt werden. Eine elektrische Durchführung von Leitungen nach außen ist nicht mehr erforderlich. Zudem kann die Auslesung des Sensorsignales über eine bestimmte Entfernung drahtlos erfolgen.

### Bezugszeichenliste

- 1: Trägerstruktur
- 2: Elektrodenanordnung
- 2o: obere Elektrode
- 2u: untere Elektrode
- 3: Kontaktdraht
- 4: Isolatorschicht
- 5,6: Elektroden
- 5u, 5o, 6u, 6o: Abschirmelektroden
- 7: zusätzliche Interdigitalelektrode
- 8: Abschirmschicht

## Patentansprüche

1. Implantierbare Vorrichtung zum Erfassen einer Dehnung einer intrakorporalen Gefäßwand mit einer an die Gefäßwand mittel- oder unmittelbar applizierbaren, flächenelastisch ausgebildeten, dielektrisches Polymer enthaltende Trägerstruktur, die wenigstens eine kapazitive Elektrodenanordnung vorsieht, deren zuordenbare elektrische Kapazität durch eine elastischen Verformung der Trägerstruktur beeinflussbar ist, und die über eine Elastizität verfügt, die mit der Elastizität der intrakorporalen Gefäßwand vergleichbar ist,
**dadurch gekennzeichnet,**
**dass** die Elektrodenanordnung wenigstens zwei jeweils aus einem elektrisch leitfähigen Polymer bestehende Elektroden vorsieht, die jeweils wenigstens einseitig einen die elektrische Kapazität der Elektrodenanordnung beeinflussenden, vollständig mit dem dielektrischen Polymer der Trägerstruktur gefüllten Zwischenraum begrenzen,
**dass** die Trägerstruktur flächig ausgebildet ist und eine Trägerstrukturober- und - unterseite aufweist, und
**dass** auf der Trägerstrukturober- und -unterseite jeweils eine Elektrode in Form einer elektrisch leitfähigen strukturierten oder unstrukturierten Polymerschicht aufgebracht ist.

2. Vorrichtung nach Anspruch 1,
**dadurch gekennzeichnet, dass** das dielektrische Polymer der Trägerstruktur ein hochelastisches Silikon ist.

3. Vorrichtung nach Anspruch 1 oder 2,
**dadurch gekennzeichnet, dass** die elektrisch leitfähigen strukturierten oder unstrukturierten Polymerschichten eine Schichtdicke aufweisen, die kleiner oder gleich als eine der Trägerstruktur zuordenbare Trägerdicke ist, durch die beide elektrisch leitfähigen Polymerschichten voneinander beabstandet sind.

4. Vorrichtung nach einem der Ansprüche 1 bis 3,
**dadurch gekennzeichnet, dass** die elektrisch leitfähigen, strukturierten oder unstrukturierten Polymerschichten in Form und Größe ähnlich oder identisch ausgebildet und in Projektion orthogonal zur Trägerstrukturober- und - unterseite zumindest teilweise überlappend angeordnet sind.

5. Vorrichtung nach einem der Ansprüche 1 bis 4,
**dadurch gekennzeichnet, dass** zumindest die an der Trägerstrukturober- und - unterseite aufgebrachten elektrisch leitfähigen Polymerschichten jeweils mit einer elastischen elektrischen Isolatorschicht bedeckt sind.

6. Implantierbare Vorrichtung zum Erfassen einer Dehnung einer intrakorporalen Gefäßwand mit einer an die Gefäßwand mittel- oder unmittelbar applizierbaren, flächenelastisch ausgebildeten, dielektrisches Polymer enthaltende Trägerstruktur, die wenigstens eine kapazitive Elektrodenanordnung vorsieht, deren zuordenbare elektrische Kapazität durch eine elastischen Verformung der Trägerstruktur beeinflussbar ist, und die über eine Elastizität verfügt, die mit der Elastizität der intrakorporalen Gefäßwand vergleichbar ist,
**dadurch gekennzeichnet,**
**dass** die Elektrodenanordnung wenigstens zwei jeweils aus einem elektrisch leitfähigen Polymer bestehende Elektroden vorsieht, die jeweils wenigstens einseitig einen die elektrische Kapazität der Elektrodenanordnung beeinflussenden, vollständig mit dem dielektrischen Polymer der Trägerstruktur gefüllten Zwischenraum begrenzen,
**dass** die Trägerstruktur flächig ausgebildet ist und eine Trägerstrukturober- und-unterseite aufweist,
**dass** in einer sich zwischen der Trägerstrukturober- und -unterseite erstreckenden und parallel zu der Trägerstrukturober- und -unterseite orientierten ersten Ebene die wenigstens zwei Elektroden vorgesehen sind, die in dieser Ebene lateral zueinander beabstandet und vollständig von dem dielektrischen Polymer der Trägerstruktur umgeben sind.

7. Vorrichtung nach Anspruch 6,
**dadurch gekennzeichnet, dass** die Elektroden in Form einer Interdigitalelektrodenstruktur ausgebildet sind, d.h. beide Elektroden weisen jeweils einen länglichen Elektrodenabschnitt auf, von dem jeweils orthogonal zu dessen Längserstreckung fingerartig ausgebildete Elektrodensegmente, so genannte Fingerelektroden abzweigen, von denen jeweils zwei längs des länglichen Elektrodenabschnittes unmittelbar benachbarte Fingerelektroden einen U-förmigen Zwischenraum mit begrenzen, in den jeweils eine Fingerelektrode der gegenüberliegenden Elektrode hineinragt.

8. Vorrichtung nach Anspruch 6 oder 7,
**dadurch gekennzeichnet, dass** in einer zweiten Ebene, die sich zwischen der Trägerstrukturober- und -unterseite erstreckt und parallel zur ersten Ebene orientiert ist und mit dieser nicht zusammenfällt, wenigstens eine weitere Elektrode vorgesehen ist, die in Form und Größe ähnlich oder identisch mit einer in der ersten Ebene befindlichen Elektrode ist und in orthogonaler Projektion zu beiden Ebenen zumindest teilweise überlappend zu dieser in der ersten Ebene befindlichen Elektrode angeordnet ist.

9. Vorrichtung nach einem der Ansprüche 1 bis 8,
**dadurch gekennzeichnet, dass** die wenigstens zwei Elektroden jeweils mit einem elektrischen Kontaktierungsmittel verbunden sind, an die wenigstens eine Induktivität zur Ausbildung eines Schwingkreises angeschlossen ist.

10. Vorrichtung nach einem der Ansprüche 1 bis 9,
**dadurch gekennzeichnet, dass** die Trägerstruktur mit der an oder innerhalb der Trägerstruktur vorgesehenen Elektroden mit einem dielektrischen Polymermaterial zumindest teilweise ummantelt ist, dessen Dielektrizitätskonstante niedriger ist als die Dielektrizitätskonstante des dielektrischen Polymers der Trägerstruktur.

11. Vorrichtung nach einem der Ansprüche 1 bis 10,
**dadurch gekennzeichnet, dass** jeder Elektrode eine Abschirmelektrode zugeordnet ist,
dass zwischen der jeweiligen Abschirmelektrode und der ihr zugeordneten Elektrode eine dielektrische Schicht vorgesehen ist, und
dass die jeweilige Abschirmelektrode an einer der Elektrodenanordnung jeweils abgewandten Seite relativ zu der ihr zugeordneten Elektrode angeordnet ist.

12. Vorrichtung nach Anspruch 11,
**dadurch gekennzeichnet, dass** die Abschirmelektrode aus einer elektrisch leitenden strukturierten oder unstrukturierten Polymerschicht besteht und über jeweils ein Verbindungsmittel elektrisch kontaktierbar ist.

13. Verfahren zur Herstellung einer implantierbare Vorrichtung zum Erfassen einer Dehnung, d.h. einer elastischen Verformung einer intrakorporalen Gefäßwand nach einem der Ansprüche 1 bis 12,
**dadurch gekennzeichnet, dass** wenigstens eine elektrisch leitfähige Polymerschicht in einem Werkstoffverbund eingebracht oder aufgebracht wird, und dass zu Zwecken der Strukturierung der elektrisch leitfähigen Polymerschicht die Polymerschicht mit einem Laserstrahl bearbeitet wird, dessen Wellenlänge von der elektrisch leitenden Polymerschicht absorbiert und von der Trägerstruktur nicht oder vernachlässigbar gering absorbiert wird.

## Claims

1. An implantable device for detecting an expansion of an intracorporeal vessel wall with a support structure which can be applied indirectly or directly to the vessel wall, is constituted two-dimensionally elastic and contains dielectric polymer, said support structure providing at least one capacitive electrode arrangement, the electrical capacitance assignable thereto being able to be influenced by an elastic deformation of the support structure, and having an elasticity which is comparable with the elasticity of the intracorporeal vessel wall,
**characterised in that**
the electrode arrangement provides at least two electrodes in each case made of an electrically conductive polymer, said electrodes each bordering at least on one side an intermediate space influencing the electrical capacitance of the electrode arrangement and being completely filled with the dielectric polymer of the support structure,
that the support structure is constituted extending two-dimensionally and comprises a support structure upper side and lower side, and
that an electrode in the form of an electrically conductive structured or unstructured polymer layer is applied respectively on the support structure upper side and lower side.

2. The device according to claim 1,
**characterised in that** the dielectric polymer of the support structure is a highly elastic silicone.

3. The device according to claim 1 or 2,
**characterised in that** the electrically conductive structured or unstructured polymer layers have a layer thickness which is less than or equal to a support thickness assignable to the support structure, by means of which support thickness the two electrically conductive polymer layers are spaced apart from one another.

4. The device according to any one of claims 1 to 3,
**characterised in that** the electrically conductive structured or unstructured polymer layers are constituted similar or identical in shape and size and are disposed at least partially overlapping in projection orthogonal to the support structure upper side and lower side.

5. The device according to any one of claims 1 to 4,
**characterised in that** at least the electrically conductive polymer layers applied on the support structure upper side and lower side are each covered with an elastic electrical insulating layer.

6. An implantable device for detecting an expansion of an intracorporeal vessel wall with a support structure which can be applied indirectly or directly to the vessel wall, is constituted two-dimensionally elastic and contains dielectric polymer, said support structure providing at least one capacitive electrode arrangement, the electrical capacitance assignable thereto being able to be influenced by an elastic deformation of the support structure, and having an elasticity which is comparable with the elasticity of the intracorporeal vessel wall,
**characterised in that**
the electrode arrangement provides at least two electrodes in each case made of an electrically conductive polymer, said electrodes each bordering at least on one side an intermediate space influencing the electrical capacitance of the electrode arrangement and being completely filled with the dielectric polymer of the support structure,
that the support structure is constituted extending two-dimensionally and comprises a support structure upper side and lower side, and
that the at least two electrodes are provided in a first plane extending between the support structure upper side and lower side and being orientated parallel to the support structure upper side and lower side, said at least two electrodes being spaced apart from one another laterally in this plane and being completely surrounded by the dielectric polymer of the support structure.

7. The device according to claim 6,
**characterised in that** the electrodes are constituted in the form of an interdigital electrode structure, i.e. the two electrodes each comprise an elongated electrode section, from which electrode segments constituted finger-like, so-called finger electrodes, in each case branch off orthogonal to the longitudinal extension of said electrode section, two of which finger electrodes directly adjacent along the elongated electrode section in each case jointly bound a U-shaped intermediate space, into which a finger electrode of the opposite electrode in each case projects.

8. The device according to claim 6 or 7,
**characterised in that** at least one further electrode is provided in a second plane, which extends between the support structure upper side and lower side and is orientated parallel to the first plane and does not coincide with the latter, said further electrode being similar or identical in shape and size to an electrode located in the first plane and, in the orthogonal projection to these two planes, being disposed at least partially overlapping with respect to this electrode located in the first plane.

9. The device according to any one of claims 1 to 8,
**characterised in that** the at least two electrodes are connected in each case to an electrical contacting means, to which at least one inductor is connected for the formation of an oscillation circuit.

10. The device according to any one of claims 1 to 9,
**characterised in that** the support structure with the electrodes provided on or inside the support structure is at least partially encased with a dielectric polymer material, the dielectric constant whereof is less than the dielectric constant of the dielectric polymer of the support structure.

11. The device according to any one of claims 1 to 10,
**characterised in that** a shielding electrode is assigned to each electrode,
that a dielectric layer is provided between the respective shielding electrode and the electrode assigned to the latter,
and that the respective shielding electrode is disposed relative to the electrode assigned to it on a side in each case facing away from the electrode arrangement.

12. The device according to claim 11,
**characterised in that** the shielding electrode comprises an electrically conductive structured or unstructured polymer layer and can be contacted electrically in each case via a connection means.

13. A method for producing an implantable device for detecting an expansion, i.e. an elastic deformation of an intracorporeal vessel wall, according to any one of claims 1 to 12,
**characterised in that** at least one electrically conductive polymer layer is introduced or applied in a material composite, and that the polymer layer is processed with a laser beam for the purpose of structuring the electrically conductive polymer layer, the wavelength of said laser beam being absorbed by the electrically conductive polymer layer and not being absorbed by the support structure or being absorbed only to a negligibly small extent by the support structure.

## Revendications

1. Dispositif implantable pour la détection d'une dilatation d'une paroi vasculaire intracorporelle avec une structure support contenant un polymère diélectrique, réalisée avec une élasticité de surface, pouvant être appliquée directement ou indirectement sur la paroi vasculaire, qui comprend au moins un agencement d'électrodes capacitif dont la capacité électrique attribuable peut être influencée par une déformation élastique de la structure support, et qui dispose d'une élasticité laquelle est comparable à l'élasticité de la paroi vasculaire intracorporelle,
**caractérisé en ce que**
l'agencement d'électrodes comprend au moins deux électrodes se composant respectivement d'un polymère à capacité électro-conductrice, lesquelles délimitent respectivement, au moins d'un côté, un espace intermédiaire complètement rempli avec le polymère diélectrique de la structure support, influençant la capacité électrique de l'agencement d'électrodes,
**en ce que** la structure support est réalisée en nappe et présente un côté supérieur et un côté inférieur de structure support, et
**en ce que** l'on applique, sur le côté supérieur et inférieur de la structure support, respectivement une électrode en forme d'une couche polymère structurée ou non structurée à capacité électro-conductrice.

2. Dispositif selon la revendication 1,
**caractérisé en ce que** le polymère diélectrique de la structure support est un silicone extrêmement élastique.

3. Dispositif selon les revendications 1 ou 2,
**caractérisé en ce que** les couches polymères structurées ou non structurées à capacité électro-conductrice présentent une épaisseur de couche, laquelle est inférieure ou égale à une épaisseur de support pouvant être attribuée à la structure support, grâce à laquelle les deux couches polymères électro-conductrices sont espacées l'une de l'autre.

4. Dispositif selon l'une des revendications 1 à 3,
**caractérisé en ce que** les couches polymères structurées ou non structurées à capacité électro-conductrice sont réalisées de manière similaire ou identique concernant la forme et la taille et sont disposées en projection orthogonalement par rapport au côté supérieur ou inférieur de la structure support de manière au moins partiellement en superposition.

5. Dispositif selon l'une des revendications 1 à 4,
**caractérisé en ce que** au moins les couches polymères électro-conductrices appliquées sur le côté supérieur et inférieur de la structure support sont respectivement recouvertes avec une couche d'isolant électrique élastique.

6. Dispositif implantable pour la détection d'une dilatation d'une paroi vasculaire intracorporelle avec une structure support contenant un polymère diélectrique, réalisée avec une élasticité de surface, pouvant être appliquée directement ou indirectement sur la paroi vasculaire, qui comprend au moins un agencement d'électrodes capacitif dont la capacité électrique attribuable peut être influencée par une déformation élastique de la structure support, et qui dispose d'une élasticité laquelle est comparable à l'élasticité de la paroi vasculaire intracorporelle,
**caractérisé en ce que**
l'agencement d'électrodes comprend au moins deux électrodes se composant respectivement d'un polymère à capacité électro-conductrice, lesquelles délimitent respectivement, au moins d'un côté, un espace intermédiaire influençant la capacité électrique de l'agencement d'électrodes, complètement rempli avec le polymère diélectrique de la structure de support,
**en ce que** la structure support est réalisée en nappe et présente un côté supérieur et un côté inférieur de structure support,
**en ce que**, dans un premier plan s'étendant entre le côté supérieur et inférieur de la structure support et orienté parallèlement au côté supérieur et inférieur de la structure support, les au moins deux électrodes sont prévues, lesquelles sont espacées latéralement l'une par rapport à l'autre dans ce plan et sont complètement entourées par le polymère diélectrique de la structure support.

7. Dispositif selon la revendication 6,
**caractérisé en ce que** les électrodes sont réalisées en forme d'une structure d'électrodes interdigitales, à savoir que les deux électrodes présentent respectivement une section d'électrode oblongue à partir de laquelle des segments d'électrodes réalisés à la manière de doigts, des dénommées électrodes en doigt, se ramifient respectivement orthogonalement à l'étendue longitudinale de celle-ci, parmi lesquelles respectivement deux de ces électrodes en doigt directement voisines le long de la section d'électrode oblongue délimitant également un espace intermédiaire en forme de U dans lequel pénètre respectivement une électrode en doigt de l'électrode située en vis-à-vis.

8. Dispositif selon les revendications 6 ou 7,
**caractérisé en ce que**, dans un deuxième plan, lequel s'étend entre le côté supérieur et inférieur de la structure support et lequel est orienté parallèlement au premier plan et ne coincide pas avec celui-ci, on prévoit au moins une autre électrode, laquelle est semblable ou identique en forme et en taille à une électrode se trouvant dans le premier plan et est disposée en projection orthogonale aux deux plans de manière au moins partiellement en superposition à cette électrode se trouvant dans le premier plan.

9. Dispositif selon l'une des revendications 1 à 8,
**caractérisé en ce que** ces au moins deux électrodes sont reliées avec un moyen de mise en contact électrique respectif, auxquels au moins une inductance pour la formation d'un circuit résonant est raccordée.

10. Dispositif selon l'une des revendications 1 à 9,
**caractérisé en ce que** la structure support, avec les électrodes prévues sur la structure support ou à l'intérieur de celle-ci, est enveloppée au moins partiellement d'une matière polymère diélectrique dont la constante diélectrique est plus faible que la constante diélectrique du polymère diélectrique de la structure support.

11. Dispositif selon l'une des revendications 1 à 10,
**caractérisé en ce que** l'on associe une électrode de blindage à chaque électrode,
**en ce que** l'on prévoit une couche diélectrique entre l'électrode de blindage respective et l'électrode qui lui est associée, et
**en ce que** l'électrode de blindage respective est disposée sur un côté tournant respectivement le dos à l'agencement d'électrodes compte tenu de l'électrode qui lui est associée.

12. Dispositif selon la revendication 11,
**caractérisé en ce que** l'électrode de blindage se compose d'une couche polymère structurée ou non structurée à capacité électro-conductrice et peut être mise en contact électrique respectivement à l'aide d'un moyen de connexion.

13. Procédé pour la fabrication d'un dispositif implantable pour la détection d'une dilatation, à savoir une déformation élastique d'une paroi vasculaire intracorporelle selon l'une des revendications 1 à 12,
**caractérisé en ce que** au moins une couche polymère à capacité électro-conductrice est noyée dans un matériau composite ou est appliquée sur celui-ci, et **en ce que**, pour les besoins de la structuration de la couche polymère à capacité électro-conductrice, on traite la couche polymère avec un faisceau laser dont la longueur d'onde est absorbée par la couche polymère électro-conductrice et n'est pas absorbée par la structure support ou bien seulement de manière négligeable.
